(19) **Europäisches Patentamt**
**European Patent Office**
**Office européen des brevets**

(11) **EP 4 148 175 A1**

(12) **EUROPEAN PATENT APPLICATION**
published in accordance with Art. 153(4) EPC

(43) Date of publication:
**15.03.2023 Bulletin 2023/11**

(21) Application number: **21837227.4**

(22) Date of filing: **05.07.2021**

(51) International Patent Classification (IPC):
**D04H 1/4266** (2012.01)    **D04H 1/498** (2012.01)
**D04H 3/16** (2006.01)    **D04H 5/03** (2012.01)

(52) Cooperative Patent Classification (CPC):
**D04H 1/4266; D04H 1/498; D04H 3/16; D04H 5/03**

(86) International application number:
**PCT/JP2021/025303**

(87) International publication number:
**WO 2022/009835 (13.01.2022 Gazette 2022/02)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.07.2020   JP 2020116943**

(71) Applicant: **Mitsui Chemicals, Inc.
Tokyo 105-7122 (JP)**

(72) Inventors:
• **FUSHIMI, Mayu
Tokyo 105-7117 (JP)**
• **ICHIKAWA, Taro
Tokyo 105-7117 (JP)**

(74) Representative: **Hoffmann Eitle
Patent- und Rechtsanwälte PartmbB
Arabellastraße 30
81925 München (DE)**

(54) **COMPOSITE NONWOVEN FABRIC AND MANUFACTURING METHOD FOR SAME**

(57)     Provided is a composite nonwoven fabric including: a silk region that contains crimped silk fibers as a main component; and a synthetic fiber nonwoven fabric region that contains polyolefin resin-containing synthetic fibers as a main component.

EP 4 148 175 A1

Processed by Luminess, 75001 PARIS (FR)

**Description**

Technical Field

The present disclosure relates to a composite nonwoven fabric and a method of producing the same.

Background Art

Nonwoven fabrics for sanitary products such as diapers and dressing bandages are used in contact with the skin and thus required to be excellent in flexibility.
As a nonwoven fabric for a sanitary product, a polyolefin-based nonwoven fabric is used in some cases.
Silk is known as a material of a nonwoven fabric. For example, Patent Document 1 discloses a medical silk nonwoven fabric consisting of only short fibers obtained by cutting silk fibers.
Further, Patent Document 2 discloses a silk nonwoven fabric that can be used as a clothing fabric or the like having a combination of a smooth texture peculiar to silk fibers and a wear resistance, which silk nonwoven fabric has a three-layer structure constituted by a silk nonwoven fabric, a mesh-like fabric, and another silk nonwoven fabric.

Patent Document 1: WO 97/07273
Patent Document 2: Japanese Patent Application Laid-Open (JP-A) No. 2003-105661

SUMMARY OF INVENTION

Technical Problem

However, according to the studies conducted by the present inventors, it was revealed that a polyolefin-based nonwoven fabric alone is sometimes insufficient in flexibility and liquid retainability.
The studies conducted by the present inventors also revealed that the silk nonwoven fabric disclosed in Patent Document 1 may be insufficient in strength since it contains only silk fibers as its fibers.
Further, the studies conducted by the present inventors revealed that the silk nonwoven fabric disclosed in Patent Document 2 may be insufficient in flexibility since a mesh-like fabric is generally rigid and has a large thickness.
An object of one aspect of the disclosure is to provide: a composite nonwoven fabric that is excellent in flexibility, strength, and liquid retainability; and a method of producing the same.

Solution to Problem

Means for solving the above-described problems encompasses the following aspects.

<1> A composite nonwoven fabric, including:

a silk region that contains crimped silk fibers as a main component; and
a synthetic fiber nonwoven fabric region that contains polyolefin resin-containing synthetic fibers as a main component.

<2> The composite nonwoven fabric according to <1>, having a thickness of 1.00 mm or less.
<3> The composite nonwoven fabric according to <1> or <2>, having a basis weight of from 20 g/cm$^2$ to 60 g/cm$^2$.
<4> The composite nonwoven fabric according to any one of <1> to <3>, wherein a content ratio of the silk fibers with respect to a total mass of the composite nonwoven fabric is from 30% by mass to 90% by mass.
<5> A spunlace nonwoven fabric, comprising the composite nonwoven fabric according to any one of <1> to <4>.
<6> The composite nonwoven fabric according to any one of <1> to <5>, having a tensile strength of 20 N/50 mm or more in a machine direction.
<7> The composite nonwoven fabric according to any one of <1> to <6>, having a tensile strength of 5 N/50 mm or more in a direction perpendicular to the machine direction in plan view.
<8> The composite nonwoven fabric according to any one of <1> to <7>, wherein the polyolefin resin contains a polypropylene resin.
<9> The composite nonwoven fabric according to any one of <1> to <8>, wherein the synthetic fibers have an average fiber diameter of from 10 μm to 30 μm.
<10> The composite nonwoven fabric according to any one of <1> to <9>, wherein the synthetic fibers contain crimped fibers.

<11> The composite nonwoven fabric according to any one of <1> to <8>, wherein the synthetic fiber nonwoven fabric region is derived from an SMS nonwoven fabric that includes two spunbond nonwoven fabrics as outermost layers and at least one melt-blown nonwoven fabric arranged between the two spunbond nonwoven fabrics.

<12> A method of producing the composite nonwoven fabric according to any one of <1> to <11>, the method including:

the step of preparing a silk fiber web that contains crimped silk fibers as a main component;
the step of preparing a synthetic fiber nonwoven fabric that contains polyolefin resin-containing synthetic fibers as a main component; and
the step of obtaining a composite nonwoven fabric by entangling the silk fiber web with the synthetic fiber nonwoven fabric.

<13> The method according to <12>, wherein, in the step of obtaining a composite nonwoven fabric, the silk fiber web with the synthetic fiber nonwoven fabric are entangled by a spunlacing process.

Advantageous Effects of Invention

According to one aspect of the disclosure, a composite nonwoven fabric that is excellent in flexibility, strength, and liquid retainability, and a method of producing the same are provided.

DETAILED DESCRIPTION OF THE INVENTION

Mode for Carrying Out the Invention

In the disclosure, those numerical ranges that are expressed with "to" each denote a range that includes the numerical values stated before and after "to" as a lower limit value and an upper limit value, respectively.

In the disclosure, when there are plural substances that correspond to a component of a composition, the indicated amount of the component in the composition means, unless otherwise specified, a total amount of the plural substances existing in the composition.

In a set of numerical ranges that are stated in a stepwise manner in the disclosure, the upper limit value or the lower limit value of a numerical range may be replaced with the upper limit value or the lower limit value of other numerical range. Further, in a numerical range stated in the disclosure, the upper limit value or the lower limit value thereof may be replaced with any relevant value indicated in any of Examples.

[Composite Nonwoven Fabric]

The composite nonwoven fabric of the disclosure includes: a silk region that contains crimped silk fibers as a main component; and a synthetic fiber nonwoven fabric region that contains polyolefin resin-containing synthetic fibers as a main component.

If necessary, the composite nonwoven fabric of the disclosure may also include other region.

The composite nonwoven fabric of the disclosure is excellent in flexibility, strength, and liquid retainability.

The reason why such an effect is obtained is presumed as follows.

The composite nonwoven fabric of the disclosure is a composite nonwoven fabric including a synthetic fiber nonwoven fabric region, and thus has a higher strength (particularly, tensile strength) than a silk fiber web that does not include the synthetic fiber nonwoven fabric region.

The composite nonwoven fabric of the disclosure is a composite nonwoven fabric including a silk region, and thus has higher flexibility and liquid retainability than a synthetic fiber nonwoven fabric that does not include the silk region.

Further, in the composite nonwoven fabric of the disclosure, the main component of the silk region is silk fibers that are crimped (hereinafter, also referred to as "crimped silk fibers"); therefore, as compared to a case where the main component of the silk region is silk fibers that are not crimped (hereinafter, also referred to as "non-crimped silk fibers"), the composite nonwoven fabric has higher shape retainability and peel strength (i.e. the strength of the composite nonwoven fabric in the thickness direction).

Specifically, with the main component of the silk region being crimped silk fibers, a silk fiber web that contains crimped silk fibers as a main component and a synthetic fiber nonwoven fabric that contains polyolefin resin-containing synthetic fibers as a main component are likely to be entangled and integrated with each other at the time of obtaining the composite nonwoven fabric of the disclosure by entangling the silk fiber web with the synthetic fiber woven fabric. As a result, the shape retainability and the peel strength (i.e. the strength in the thickness direction) of the resulting composite nonwoven fabric are improved.

It is noted here that the term "web" in the silk fiber web refers to a fabric in general (e.g., a nonwoven fabric or a woven fabric).

The term "main component" used herein refers to a component having the largest mass content among all components that are contained.

The composite nonwoven fabric of the disclosure may include only one silk region, or two or more silk regions.

The composite nonwoven fabric of the disclosure may include only one synthetic fiber nonwoven fabric region, or two or more synthetic fiber nonwoven fabric regions.

A preferred aspect of the composite nonwoven fabric of the disclosure is an aspect in which at least one silk region and at least one synthetic fiber nonwoven fabric region are arranged in the thickness direction of the composite nonwoven fabric.

From the standpoint of the touch feel of the composite nonwoven fabric, a more preferred aspect of the composite nonwoven fabric of the disclosure is an aspect A of including:

two silk regions arranged in the thickness direction of the composite nonwoven fabric; and

at least one synthetic fiber nonwoven fabric region arranged between the two silk regions.

The composite nonwoven fabric according to the aspect A may further include one or more silk regions in addition to the two silk regions, as long as the above-described requirement of the aspect A is satisfied. For example, the aspect A also encompasses an aspect in which silk regions and synthetic fiber nonwoven fabric regions are alternately arranged and a total number of the silk regions is three or more, as long as the requirement of the aspect A is satisfied.

The composite nonwoven fabric of the disclosure is preferably produced by entangling a silk fiber web that contains crimped silk fibers as a main component with a synthetic fiber nonwoven fabric that contains polyolefin resin-containing synthetic fibers as a main component.

As a result, a silk region is formed as a region derived from the silk fiber web, and a synthetic fiber nonwoven fabric region is formed as a region derived from the synthetic fiber nonwoven fabric, whereby the composite nonwoven fabric of the disclosure is produced.

The composite nonwoven fabric of the disclosure is preferably a spunlace nonwoven fabric.

The term "spunlace nonwoven fabric" used herein refers to a nonwoven fabric obtained by entangling a silk fiber web that contains crimped silk fibers as a main component with a synthetic fiber nonwoven fabric that contains polyolefin resin-containing synthetic fibers as a main component through a spunlacing process (i.e. a hydroentanglement process).

When the composite nonwoven fabric of the disclosure is a spunlace nonwoven fabric, the softness of silk fibers is more effectively maintained in the composite nonwoven fabric.

Preferred physical properties of the composite nonwoven fabric will now be described.

With regard to the measurement conditions of the following preferred physical properties, reference can be made to the descriptions in the section of Examples.

<Thickness of Composite Nonwoven Fabric>

The composite nonwoven fabric of the disclosure has a thickness of preferably 1.00 mm or less, more preferably from 0.20 mm to 1.00 mm, still more preferably from 0.20 mm to 0.80 mm, particularly preferably from 0.25 mm to 0.60 mm.

When the thickness of the composite nonwoven fabric is 1.00 mm or less, for example, by using the composite nonwoven fabric as a sanitary material, the thickness of the sanitary material can be reduced. Further, when the thickness of the composite nonwoven fabric is 1.00 mm or less, the composite nonwoven fabric tends to have a higher flexibility. The composite nonwoven fabric having a thickness of 1.00 mm or less can be obtained by, for example, composite formation through a spunlacing process as described below.

<Basis Weight of Composite Nonwoven Fabric>

The composite nonwoven fabric of the disclosure has a basis weight of preferably from 20 g/cm$^2$ to 60 g/cm$^2$, more preferably from 20 g/cm$^2$ to 50 g/cm$^2$. When the basis weight of the composite nonwoven fabric is in this range, the strength and the thinness tend to be both satisfied at the same time, which is preferred.

In the disclosure, the basis weight of the composite nonwoven fabric is a value obtained by dividing the mass of the composite nonwoven fabric by the area of the composite nonwoven fabric.

In the disclosure, the thickness and the basis weight of the composite nonwoven fabric are each measured using one or more samples collected from the composite nonwoven fabric. A total area of the samples used for the measurement is set at 250 cm$^2$ or larger (e.g., 500 cm$^2$).

<Content Ratio of Silk Fibers>

In the composite nonwoven fabric of the disclosure, a content ratio of silk fibers (particularly, a total content ratio of crimped silk fibers and non-crimped silk fibers) is preferably from 30% by mass to 90% by mass, more preferably from 35% by mass to 80% by mass, still more preferably from 40% by mass to 70% by mass, with respect to a total mass of the composite nonwoven fabric.

When the content of crimped silk fibers is 30% by mass or more, the composite nonwoven fabric tends to have higher flexibility and liquid retainability, which is preferred.

When the content of crimped silk fibers is 90% by mass or less, the composite nonwoven fabric tends to have a higher strength, which is also preferred.

<Tensile Strength of Composite Nonwoven Fabric>

The composite nonwoven fabric of the disclosure has a tensile strength of preferably 20 N/50 mm or more in a machine direction (MD) (this tensile strength is also hereinafter referred to as "MD tensile strength").

The MD tensile strength of the composite nonwoven fabric is more preferably from 20 N/50 mm to 70 N/50 mm, still more preferably from 20 N/50 mm to 50 N/50 mm.

In the disclosure, the unit "N/50 mm" means the tensile strength (N) in the longitudinal direction that is measured using a sample of 50 mm in width.

In the disclosure, the term "MD" in the MD (i.e. machine direction; the same applies below) tensile strength of the composite nonwoven fabric means the MD of the synthetic fiber nonwoven fabric region. In other words, the MD tensile strength of the composite nonwoven fabric means the tensile strength of the composite nonwoven fabric in the MD of the synthetic fiber nonwoven fabric region.

The composite nonwoven fabric of the disclosure has a tensile strength of preferably 5 N/50 mm or more in a direction (cross direction: CD) perpendicular to the machine direction in plan view (this tensile strength is also referred to as "CD tensile strength").

The CD tensile strength of the composite nonwoven fabric is more preferably from 5 N/50 mm to 30 N/50 mm, still more preferably from 5 N/50 mm to 20 N/50 mm.

In the disclosure, the term "CD" in the CD (i.e. the direction perpendicular to the machine direction in plan view; the same applies below) tensile strength of the composite nonwoven fabric means the CD of the synthetic fiber nonwoven fabric region. In other words, the CD tensile strength of the composite nonwoven fabric means the tensile strength of the composite nonwoven fabric in the CD of the synthetic fiber nonwoven fabric region.

Methods of measuring the MD tensile strength and the CD tensile strength of the composite nonwoven fabric are as described below in the section of Examples. It is noted here, however, that the length of a test piece is not limited to be 200 mm as in Examples, and may be any length (e.g., 120 mm or more) as long as a chuck distance of 100 mm can be ensured.

<Air Permeability of Composite Nonwoven Fabric>

The composite nonwoven fabric of the disclosure has an air permeability ($cm^3/cm^2/sec$; hereinafter, also denoted as "ccs") of preferably from 50 ccs to 300 ccs, more preferably from 80 ccs to 200 ccs.

The air permeability is preferably 50 ccs or more since this tends to prevent the skin from being sweaty when the composite nonwoven fabric is used as a sanitary material.

The air permeability is also preferably 300 ccs or less since this tends to further improve the liquid retainability of the composite nonwoven fabric.

The air permeability of the composite nonwoven fabric is measured using one or more samples collected from the composite nonwoven fabric. A total area of the samples used for the measurement is set at 10,000 $mm^2$ or larger (e.g., 22,500 $mm^2$).

<Peel Strength of Composite Nonwoven Fabric>

The composite nonwoven fabric of the disclosure has a peel strength of preferably not less than 0.3 N, more preferably from 0.5 N to 2.0 N, still more preferably from 0.6 N to 1.5 N.

A method of measuring the peel strength of the composite nonwoven fabric is as described below in the section of Examples.

<Bending Resistance of Composite Nonwoven Fabric>

The composite nonwoven fabric has an MD bending resistance of preferably from 35 mm to 60 mm, more preferably from 40 mm to 50 mm.

The composite nonwoven fabric has a CD bending resistance of preferably from 20 mm to 30 mm, more preferably from 21 mm to 16 mm.

Methods of measuring the MD bending resistance and the CD bending resistance of the composite nonwoven fabric are as described below in the section of Examples.

Preferred aspects of the silk region and the synthetic fiber nonwoven fabric will now be described.

<Silk Region>

The silk region is a region that contains crimped silk fibers as a main component.

The silk region is preferably formed using a silk fiber web (e.g., a silk fiber nonwoven fabric or a silk fiber woven fabric) that contains crimped silk fibers as a main component.

The crimped silk fibers are fibers obtained by crimping non-crimped silk fibers (i.e. (i.e. silk that is not crimped).

Any known method can be applied as a method of crimping the non-crimped silk fibers.

Examples of the method of crimping the non-crimped silk fibers include:

a gear crimping method in which the non-crimped silk fibers are passed between a pair of grooved rollers fitting each other like gears and thereby shaped into a zigzag form; and
a buckling method in which, using a funnel-like jig including a tubular portion, the non-crimped silk fibers in the state of being bent in a zigzag shape are pushed into the tubular portion of the jig, and subsequently pushed out of the tubular portion and thereby flexed.

With regard to the non-crimpled silk fibers, the method of crimping the non-crimped silk fibers, and the crimped silk fibers, known technologies can be applied with no particular limitation, and reference can be made to known documents such as JP-A No. 2003-105661  and JP-A No. 2006-207069 as appropriate.

The silk region may also contain other components in addition to the crimped silk fibers.

Examples of the other components include non-crimped silk fibers, and fibers other than silk fibers.

When the composite nonwoven fabric is produced by entangling a silk fiber web with a synthetic fiber nonwoven fabric, synthetic fibers may be incorporated into the silk region derived from the silk fiber web due to the entangling.

The content ratio of the crimped silk fibers is preferably not less than 50% by mass, more preferably not less than 60% by mass, still more preferably not less than 80% by mass, particularly preferably not less than 90% by mass, with respect to a total mass of the silk region.

The crimped silk fibers contained in the silk region have an average fiber diameter of preferably from 2 $\mu$m to 50 $\mu$m, more preferably from 3 $\mu$m to 30 $\mu$m, still more preferably 5 $\mu$m to 20 $\mu$m.

When the average fiber diameter of the crimped silk fibers is in this range, entanglements thereof with the synthetic fibers are formed more appropriately, so that the peel strength of the composite nonwoven fabric tends to be further improved, which is preferred.

The average fiber diameter refers to an arithmetic mean value of the diameters of 30 crimped silk fibers.

The crimped silk fibers contained in the silk region have an average fiber length of preferably from 10 mm to 100 mm, more preferably from 20 mm to 80 mm, still more preferably from 30 mm to 70 mm.

The average fiber length refers to an arithmetic mean value of the lengths of 30 crimped silk fibers.

Further, the fiber length of each crimped silk fiber is the fiber length measured in a state where the crimped shape is straightened out.

From the standpoint of further improving the shape retainability and the peel strength of the composite nonwoven fabric, the crimped silk fibers have a crimp pitch of preferably from 0.2 mm to 3.0 mm, more preferably from 0.5 mm to 2.0 mm.

When the crimped silk fibers have a zigzag shape of repeating peaks and valleys, the term "crimp pitch" used herein refers to an average distance between the tops of adjacent peaks.

The average distance refers to an arithmetic mean of values (distance) measured at 30 spots.

From the standpoint of further improving the shape retainability and the peel strength of the composite nonwoven fabric, the crimped silk fibers have a crimp degree of preferably 3% or higher, more preferably 5% or higher, still more preferably 10% or higher, particularly preferably 15% or higher.

An upper limit of the crimp degree of the crimped silk fibers is preferably 80%, more preferably 50%, still more preferably 40%.

The term "crimp degree" used herein refers to the degree of crimping. The larger the value of the crimp degree, the higher is the degree of crimping. A method of calculating the crimp ratio will be described below in the section of Examples.

<Synthetic Fiber Nonwoven Fabric Region>

The synthetic fiber nonwoven fabric region is a region that contains a synthetic fiber nonwoven fabric.

The synthetic fiber nonwoven fabric region is preferably formed using a synthetic fiber nonwoven fabric.

The synthetic fiber nonwoven fabric region and its raw material that is a synthetic fiber nonwoven fabric both contain polyolefin resin-containing synthetic fibers as a main component.

The content ratio of the synthetic fibers with respect to a total mass of the synthetic fiber nonwoven fabric region is preferably not less than 50% by mass, more preferably not less than 60% by mass, still more preferably not less than 80% by mass, particularly preferably not less than 90% by mass.

In the same manner, the content ratio of the synthetic fibers with respect to a total mass of the synthetic fiber nonwoven fabric is preferably not less than 50% by mass, more preferably not less than 60% by mass, still more preferably not less than 80% by mass, particularly preferably not less than 90% by mass.

The synthetic fibers may contain only one kind of polyolefin resin, or two or more kinds of polyolefin resins.

The content ratio of the polyolefin resin (total content ratio when two or more kinds of polyolefin resins are contained) in the synthetic fibers is preferably not less than 50% by mass, more preferably not less than 60% by mass, still more preferably not less than 80% by mass, particularly preferably not less than 90% by mass, with respect to a total mass of the synthetic fibers.

The term "polyolefin resin" used herein refers to a resin that contains an olefin unit (i.e. a structural unit formed by polymerization of olefin) as a main component.

The content ratio of the olefin unit with respect to a total mass of the polyolefin resin is preferably not less than 50% by mass, more preferably not less than 60% by mass, still more preferably not less than 80% by mass, particularly preferably not less than 90% by mass.

The polyolefin resin may also contain a structural unit other than the olefin unit.

The polyolefin resin may contain only one kind of olefin unit, or two or more kinds of olefin units.

In other words, the polyolefin resin may be a homopolymer of one kind of olefin, or a copolymer of two or more kinds of olefins.

Examples of the olefin forming the olefin unit include $\alpha$-olefins such as ethylene, propylene, 1-butene, 1-pentene, 1-hexene, 1-octene, 1-decene, 3-methyl-1-butene, 3-methyl-1-pentene, 3-ethyl-1-pentene, 4-methyl-1-pentene, and 4-methyl-1-hexene.

From the standpoint of the balance between the strength and the flexibility, the polyolefin resin preferably contains a polypropylene resin.

In this case, the polyolefin resin may contain only one kind of polypropylene resin, or two or more kinds of polypropylene resins.

The term "polypropylene resin" used herein refers to a resin that contains a propylene unit (i.e. a structural unit formed by polymerization of propylene) as a main component.

The content ratio of the propylene unit with respect to a total mass of the polypropylene resin is preferably not less than 50% by mass, more preferably not less than 60% by mass, still more preferably not less than 80% by mass, particularly preferably not less than 90% by mass.

The polypropylene resin may also contain a structural unit other than the propylene unit (preferably a structural unit derived from an $\alpha$-olefin other than propylene).

The polypropylene resin has a melt flow rate (MFR) (particularly, MFR measured in accordance with ASTM D1238 at 230°C and a load of 2.16 kg; the same applies below) of preferably from 1 g/10 min to 3,000 g/10 min, more preferably from 2 g/10 min to 2,000 g/10 min, still more preferably from 10 g/10 min to 1,000 g/10 min, particularly preferably from 10 g/10 min to 700 g/10 min.

The polypropylene resin that may be contained in the polyolefin resin is preferably at least one of a propylene homopolymer and a propylene-$\alpha$-olefin copolymer (i.e. a copolymer of propylene and an $\alpha$-olefin other than propylene).

The propylene-$\alpha$-olefin copolymer may contain only one kind of $\alpha$-olefin (i.e. $\alpha$-olefin other than propylene), or two or more kinds of $\alpha$-olefins.

The $\alpha$-olefin (i.e. $\alpha$-olefin other than propylene) in the propylene-$\alpha$-olefin copolymer is preferably an $\alpha$-olefin having from 2 to 10 carbon atoms other than propylene (e.g., ethylene, 1-butene, 1-pentene, 1-hexene, 1-octene, 1-decene, 3-methyl-1-butene, 3-methyl-1-pentene, 3-ethyl-1-pentene, 4-methyl-1-pentene, or 4-methyl-1-hexene).

The propylene-$\alpha$-olefin copolymer is preferably a propylene-ethylene copolymer, more preferably a propylene-ethylene random copolymer.

The $\alpha$-olefin content in the propylene-$\alpha$-olefin copolymer (i.e. the amount (% by mole) of $\alpha$-olefin unit with respect to all structural units of the propylene-$\alpha$-olefin copolymer) is preferably from 1% by mole to 40% by mole, more preferably from 2% by mole to 30% by mole, still more preferably from 2% by mole to 20% by mole, particularly preferably from 2% by mole to 10% by mole.

The propylene content in the propylene-$\alpha$-olefin copolymer (i.e. the amount (% by mole) of propylene unit with respect to all structural units of the propylene-$\alpha$-olefin copolymer) is preferably from 60% by mole to 99% by mole, more preferably from 70% by mole to 98% by mole, still more preferably from 80% by mole to 98% by mole, particularly preferably from 90% by mole to 98% by mole.

The propylene homopolymer has a melting point of preferably 155°C or higher, more preferably from 157°C to 165°C.

The propylene-$\alpha$-olefin copolymer has a melting point of preferably from 120°C to 154°C, more preferably from 130°C to 150°C.

When the polyolefin resin contains a polypropylene resin, the polyolefin resin may also contain other resin in addition to the polypropylene resin.

When the polyolefin resin contains a polypropylene resin, the content ratio of the polypropylene resin with respect to a total mass of the polyolefin resin is preferably not less than 50% by mass, more preferably not less than 60% by mass, still more preferably not less than 80% by mass, particularly preferably not less than 90% by mass.

(Average Fiber Diameter)

From the standpoint of further facilitating the entanglement of the synthetic fibers and the crimped silk fibers and thereby further improving the peel strength, the synthetic fibers have an average fiber diameter of preferably from 10 $\mu$m to 30 $\mu$m.

A method of measuring the average fiber diameter of the synthetic fibers is as described below in the section of Examples.

The above-described average fiber diameter is likely to be realized when a spunbond nonwoven fabric is used as the synthetic fiber nonwoven fabric for the formation of the synthetic fiber nonwoven fabric region.

(Crimped Fibers)

From the standpoint of further facilitating the entanglement of the synthetic fibers and the crimped silk fibers and thereby further improving the peel strength, the synthetic fibers preferably contain crimped fibers.

In the disclosure, when the synthetic fibers contain crimped fibers, the "crimped fibers" refer to at least either of irregular-shaped fibers (i.e. fibers having an asymmetrical cross-section) and composite fibers containing two or more synthetic resins (i.e. crimped composite fibers).

The crimped fibers are preferably crimped composite fibers.

The crimped composite fibers preferably contain two or more kinds of polyolefin resins (preferably two or more kinds of polypropylene resins) that are different in at least one of composition, molecular weight, and physical properties (e.g., crystallization temperature, melting point, softening point, crystallization rate, and melt viscosity).

The crimped composite fibers are preferably eccentric core-sheath crimped composite fibers.

The "eccentric core-sheath crimped composite fibers" refer to crimped composite fibers which are each formed of a core portion and a sheath portion surrounding at least a part of the core portion at a fiber transverse cross-section (i.e. a cross-section perpendicular to the fiber length), and in which the center of the core portion and that of the sheath portion do not overlap with each other.

The eccentric core-sheath crimped composite fibers preferably have a side-by-side type transverse cross-sectional shape.

The term "side-by-side type transverse cross-sectional shape" used herein refers to a shape in which the sheath portion surrounds only a part of the core portion and the remainder of the core portion is exposed to the outside.

The crimped composite fibers (e.g., eccentric core-sheath crimped composite fibers) are not particularly limited, and known crimped composite fibers disclosed in a known document such as Japanese Patent No. 4009196, Japanese Patent No. 5139669, or Japanese Patent No. 5289459 can be used.

The crimped composite fibers are preferably eccentric core-sheath crimped composite fibers each including: a core portion that contains a first polyolefin resin; and a sheath portion that contains a second polyolefin resin different from the first polyolefin resin.

In the eccentric core-sheath crimped composite fibers, the mass ratio of the core portion with respect to the sheath portion (hereinafter, also referred to as "mass ratio [core/sheath]") is preferably from 0.05 to 5.00, more preferably from 0.10 to 1.00, still more preferably from 0.10 to 0.50.

The first polyolefin resin contained in the core portion is preferably a propylene resin, more preferably a propylene homopolymer.

The second polyolefin resin contained in the sheath portion is preferably a propylene resin, more preferably a

propylene-$\alpha$-olefin copolymer.

Preferred aspects of the propylene homopolymer and the propylene-$\alpha$-olefin copolymer are as described above.

A value obtained by subtracting the melting point of the second polyolefin resin from the melting point of the first polyolefin resin (i.e. melting point difference [(melting point of first polyolefin resin) - (melting point of second polyolefin resin)]) is preferably larger than 0°C, more preferably 5°C or larger, still more preferably 10°C or larger, particularly preferably 15°C or larger.

An upper limit of the melting point difference [(melting point of first polyolefin resin) - (melting point of second polyolefin resin)] is preferably 50°C, more preferably 40°C, still more preferably 30°C.

The crimped fibers have an average fiber diameter of preferably from 10 $\mu$m to 30 $\mu$m, more preferably from 10 $\mu$m to 20 $\mu$m.

This average fiber diameter is likely to be realized when a spunbond nonwoven fabric is used as the synthetic fiber nonwoven fabric for the formation of the synthetic fiber nonwoven fabric region containing the crimped fibers.

Examples of a preferred aspect of the composite nonwoven fabric of the disclosure include an aspect in which the synthetic fiber nonwoven fabric region is a region derived from a spunbond nonwoven fabric (more preferably a spunbond nonwoven fabric containing crimped fibers).

(Region Derived from SMS Nonwoven Fabric)

Examples of a preferred aspect of the composite nonwoven fabric of the disclosure also include an aspect in which the synthetic fiber nonwoven fabric region is a region derived from an SMS nonwoven fabric.

The term "SMS nonwoven fabric" used herein refers to a layered nonwoven fabric that includes two spunbond nonwoven fabrics as outermost layers, and at least one melt-blown nonwoven fabric arranged between the two spunbond nonwoven fabrics.

According to this preferred aspect, the entanglement of the synthetic fibers and the crimped silk fibers is further facilitated by the effect of the melt-blown nonwoven fabric in the SMS nonwoven fabric, as a result of which the peel strength is further improved.

The SMS nonwoven fabric may include only one melt-blown nonwoven fabric, or two or more melt-blown nonwoven fabrics, between the two spunbond nonwoven fabrics constituting the outermost layers.

The SMS nonwoven fabric may also include other spunbond nonwoven fabric between the two spunbond nonwoven fabrics constituting the outermost layers. The SMS nonwoven fabric may have a structure of spunbond nonwoven fabric/spunbond nonwoven fabric/melt-blown nonwoven fabric/spunbond nonwoven fabric/spunbond nonwoven fabric, or spunbond nonwoven fabric/spunbond nonwoven fabric/melt-blown nonwoven fabric/spunbond nonwoven fabric.

The spunbond nonwoven fabrics in the SMS nonwoven fabric have an average fiber diameter of, for example, from 10 $\mu$m to 30 $\mu$m.

The melt-blown nonwoven fabric in the SMS nonwoven fabric has an average fiber diameter of, for example, from 0.1 $\mu$m to 7 $\mu$m.

Methods of measuring the average fiber diameter of the spunbond nonwoven fabrics in the SMS nonwoven fabric and that of the melt-blown nonwoven fabric in the SMS nonwoven fabric are as described below in the section of Examples.

Preferred aspects of the fibers contained in the spunbond nonwoven fabrics of the SMS nonwoven fabric and the fibers contained in the melt-blown nonwoven fabric of the SMS nonwoven fabric are as described above for the polyolefin resin-containing synthetic fibers.

(Use)

Examples of preferred use of the composite nonwoven fabric of the disclosure include: hygienic materials, such as diapers and sanitary products; general cosmetic products, such as masks, face masks, makeup remover sheets, and cosmetic pads; general medical sheets, such as wound dressings and surgical pads; cell culture substrates; apparel products; wig substrates; beddings; food packaging materials; and electronic materials. Thereamong, the composite nonwoven fabric of the disclosure can be preferably used in the face mask applications where flexibility, strength, and liquid retainability are required, particularly as the skin-contacting surface of a face mask.

[One Example of Method of Producing Composite Nonwoven Fabric]

One example of a method of producing the composite nonwoven fabric of the disclosure (hereinafter, referred to as "production method A") includes:

the step of preparing a silk fiber web that contains crimped silk fibers as a main component;

the step of preparing a synthetic fiber nonwoven fabric that contains polyolefin resin-containing synthetic fibers as a main component; and

the step of obtaining a composite nonwoven fabric by entangling the silk fiber web with the synthetic fiber nonwoven fabric.

In the production method A, since a silk fiber web containing crimped silk fibers as a main component is used, the silk fiber web can be effectively entangled with the synthetic fiber nonwoven fabric.

Therefore, the composite nonwoven fabric of the disclosure can be produced in a preferred manner.

With regard to a preferred aspect of the silk fiber web prepared in the step of preparing a silk fiber web, reference can be made to the above descriptions of the silk region in the composite nonwoven fabric as appropriate.

The step of preparing a silk fiber web may be the step of merely preparing a silk fiber web produced in advance, or may be the step of producing a silk fiber web for carrying out the production method A.

In the step of preparing a silk fiber web, only one silk fiber web may be prepared, or plural silk fiber webs may be prepared. The number of silk fiber webs can be selected as appropriate in accordance with the number of silk fiber webs to be used in the step of obtaining a composite nonwoven fabric.

With regard to a preferred aspect of the synthetic fiber nonwoven fabric prepared in the step of preparing a synthetic fiber nonwoven fabric, reference can be made to the above descriptions of the synthetic fiber nonwoven fabric region in the composite nonwoven fabric as appropriate.

In the production method A, from the standpoint of further facilitating the entanglement of the synthetic fibers and the crimped silk fibers and consequently further improving the peel strength of the resulting composite nonwoven fabric, the synthetic fiber nonwoven fabric prepared in the step of preparing a synthetic fiber nonwoven fabric is preferably a spunbond nonwoven fabric (preferably a spunbond nonwoven fabric containing crimped fibers) or an SMS nonwoven fabric.

The step of preparing a synthetic fiber nonwoven fabric may be the step of merely preparing a synthetic fiber nonwoven fabric produced in advance, or may be the step of producing a synthetic fiber nonwoven fabric for carrying out the production method A.

In the step of preparing a synthetic fiber nonwoven fabric, only one synthetic fiber nonwoven fabric may be prepared, or plural synthetic fiber nonwoven fabrics may be prepared. The number of synthetic fiber nonwoven fabrics can be selected as appropriate in accordance with the number of synthetic fiber nonwoven fabrics to be used in the step of obtaining a composite nonwoven fabric.

The step of obtaining a composite nonwoven fabric is the step of entangling at least one silk fiber web with at least one synthetic fiber nonwoven fabric to obtain a composite nonwoven fabric.

By this step, the silk fiber web with the synthetic fiber nonwoven fabric are entangled three-dimensionally and thereby integrated, as a result of which a composite nonwoven fabric that includes a silk region derived from the silk fiber web with a synthetic fiber nonwoven fabric region derived from the synthetic fiber nonwoven fabric is obtained.

In the step of obtaining a composite nonwoven fabric, it is preferred to entangle two silk fiber webs as outermost layers and at least one synthetic fiber nonwoven fabric arranged between the two silk fiber webs.

In this aspect, the silk fiber webs and the synthetic fiber nonwoven fabric may be alternately arranged.

The silk fiber webs and the synthetic fiber nonwoven fabric are preferably entangled by a spunlacing process. When the silk fiber webs and the synthetic fiber nonwoven fabric are entangled by a spunlacing process, the softness of silk fibers is maintained more effectively in the resulting composite nonwoven fabric.

The "spunlacing process", which is also called "hydroentanglement process", is a process of spraying water stream to a laminated body of the silk fiber webs and the synthetic fiber nonwoven fabric from both the upper surface side and the lower surface side of the laminated body and thereby entangling the silk fiber webs and the synthetic fiber nonwoven fabric.

The pressure of the water stream is preferably from 0.5 MPa to 10 MPa, more preferably from 1 MPa to 8 MPa, still more preferably from 1 MPa to 6 MPa.

The spraying of water stream may be performed continuously or intermittently.

In the spunlacing process, the conditions of the sprayed water stream and the number of spray operations on the upper surface side may be the same as or different from those on the lower surface side.

With regard to the spunlacing process (hydroentanglement process), reference can be made to, for example, the process described in a known document such as WO 2020/036143. Examples

Examples of the disclosure will now be described; however, the disclosure is not limited to the below-described Examples.

[Example 1]

<Production of Composite Nonwoven Fabric>

(Production of Silk Fiber Webs (First Layer and Third Layer))

Commercially available silk fibers (A1 SLIVER, average fiber diameter = 12 $\mu$m, manufactured by Hasegawa Corporation Co., Ltd.) were subjected to two mechanical crimping operations using a jig to obtain a bundle of crimped silk fibers (i.e. silk fibers that were crimped; denoted as "crimped silk" in Table 1).

It is noted here that the average fiber diameter of the silk fibers refers to an average value of the diameters of 30 silk fibers. The fiber diameter of each silk fiber was measured under a scanning electron microscope (SU3500 manufactured by Hitachi, Ltd.; at a magnification of $\times$300).

As the jig in the mechanical crimping operations, a funnel-like jig including a tubular portion was used.

Each mechanical crimping operation consisted of pushing the silk fibers into the tubular portion of the jig with the silk fibers being bent in a zigzag shape, and subsequently pushing the silk fibers out of the tubular portion in the form of a bundle.

In the thus obtained bundle of crimped silk fibers, the crimped silk fibers had a zigzag shape of repeating peaks and valleys. In this zigzag shape, the crimp pitch, namely the average distance between the tops of adj acent peaks (particularly, the average of the values measured at 30 spots), was 1.0 mm.

Next, the bundle of the crimped silk fibers was cut at an average fiber length of 51 mm.

It is noted here that the average fiber length of the crimped silk fibers refers to an average value of the lengths of 30 crimped silk fibers.

Using the thus cut bundle of the crimped silk fibers as a raw material, crimped silk fiber webs (particularly, crimped silk fiber nonwoven fabrics; the same applies below) were produced using a fiber opening machine and a roller-type parallel carding machine.

By the above-described operations, a crimped silk fiber web having a basis weight of 13 g/m$^2$ and a crimped silk fiber web having a basis weight of 14 g/m$^2$ were obtained as a first layer and a third layer, respectively.

It is noted here that the basis weight was modified by changing the rotation speed of the roller-type parallel carding machine. A method of measuring the basis weight will be described below.

(Production of Synthetic Fiber Nonwoven Fabric (Second Layer))

The followings were each prepared as raw materials:

a propylene homopolymer having a melting point of 162°C and an MFR (measured in accordance with ASTM D1238 at a temperature of 230°C and a load of 2.16 kg; the same applies hereinafter unless otherwise specified) of 60 g/10 min; and
a propylene-ethylene random copolymer having a melting point of 142°C, an MFR of 60 g/10 min, an ethylene unit content of 4.0% by mole, and a propylene unit content of 96.0% by mole.

Using these raw materials, a synthetic fiber nonwoven fabric (a spunbond nonwoven fabric in Example 1; denoted as "SB (single layer)" in Table 1) was produced as a second layer by a spunbonding method.

In more detail, crimped composite fibers (particularly, eccentric core-sheath crimped composite fibers), in which the core portion was formed of a propylene homopolymer and the sheath portion was formed of a propylene-ethylene random copolymer and which had a mass ratio [core portion/sheath portion] of 0.25, were obtained by composite melt spinning, and the thus obtained crimped composite fibers were deposited on a collection surface, after which the resultant was embossed at an embossing area ratio of 18% and an embossing temperature of 110°C, whereby a synthetic fiber nonwoven fabric (spunbond nonwoven fabric) having a basis weight of 17 g/m$^2$ was obtained as the second layer.

In the thus obtained synthetic fiber nonwoven fabric, the crimped composite fibers had an average fiber diameter of 16 $\mu$m.

The crimped composite fibers (particularly, eccentric core-sheath crimped composite fibers) had a side-by-side type cross-sectional shape.

From the synthetic fiber nonwoven fabric obtained as the second layer, 10 rectangular test pieces of 10 mm (length) $\times$ 10 mm (width) were collected. The thus collected test pieces were each observed under an ECLIPSE E400 microscope manufactured by Nikon Corporation at a magnification of $\times$300. In this observation, 20 crimpled composite fibers of 9 $\mu$m or more in fiber diameter were selected for each test piece, and the fiber diameter was measured to the first decimal place in micrometers ($\mu$m) for each of the thus selected crimpled composite fibers of 9 $\mu$m or more in fiber diameter. The average of the thus obtained 200 measured values was calculated and defined as the average fiber diameter of the crimped composite fibers.

(Production of Composite Nonwoven Fabric)

The above-obtained first layer, second layer, and third layer were disposed on one another in this order to obtain a laminated body, and this laminated body was placed on a net. In this process, the layers were disposed on one another such that the machine direction (MD) and that of the third layer were each oriented substantially parallel to the MD of the second layer.

Next, the net and the laminated body placed thereon were advanced at a speed of 7 m/min to perform a spunlacing process (i.e. hydroentanglement process) on the laminated body.

The thus spunlaced laminated body was sequentially subjected to a surface smoothing treatment using a nip roll, a drying treatment at 110°C using a hot air penetration-type heating machine, and another surface smoothing treatment using a nip roll in this order to obtain a composite nonwoven fabric as a spunlace nonwoven fabric.

The spunlacing process (i.e. hydroentanglement process) was performed by spraying the upper surface of the laminated body once with each of a columnar water jet having a water pressure of 2 MPa, a columnar water jet having a water pressure of 4 MPa, and a columnar water jet having a water pressure of 4 MPa, and subsequently spraying the lower surface of the laminated body once with a columnar water jet having a water pressure of 4 MPa, using a water-jet apparatus equipped with a nozzle having 0.10-mm orifices formed at 1-mm intervals. The distance between the respective surface of the laminated body and the orifices was set at 15 mm.

<Measurements and Evaluations>

For the above-produced composite nonwoven fabric and its materials, the following measurements and evaluations were performed.

The results thereof are shown in Table 1.

(1) Basis Weight (g/m$^2$; "gsm" in Table 1)

The basis weight of the composite nonwoven fabric was determined by collecting five 10 cm-square samples from the composite nonwoven fabric, and dividing the total mass of the thus collected five samples by the total area of the five samples.

The basis weight of each of the first to the third layers was determined by the same method as the basis weight (g/m$^2$) of the composite nonwoven fabric.

(2) Crimp Degree of Crimped Silk Fibers

From a bundle of the crimped silk fibers cut at an average fiber length of 51 mm, a bundle X1 of the crimped silk fibers was recovered in an amount that the recovered fiber bundle had a cross-sectional area of about 1 mm$^2$.

The thus recovered bundle X1 of the crimped silk fibers was arranged along a ruler as non-undulating as possible. One end of the bundle X1 was held with forceps, and the bundle X1 was immobilized on the side of the other end at a position 3.0 cm away from the position being held with the forceps.

Subsequently, the forceps was pulled by hand toward one end of the bundle X1 at a rate of 0.5 cm/sec to elongate the portion of the bundle X1 between the immobilized position and the position held with the forceps (i.e. the portion having a pre-elongation length of 3.0 cm). This operation was performed until the forceps could no longer be pulled by hand (i.e. until the bundle X1 could no longer be elongated) and, at this point, the length (cm) of the portion between the immobilized position and the position held with the forceps (hereinafter, referred to as "elongated length") was measured. This operation was repeated 10 times, and the average of the thus obtained 10 measured values was calculated and defined as "average elongated length (cm)".

From the pre-elongation length of 3.0 cm and the average elongated length, the crimp degree (%) was calculated by the following equation:

$$\text{Crimp degree (\%)} = \{1 - (3.0/\text{average elongated length (cm)})\} \times 100$$

(3) Silk Content Ratio (% by mass) in Composite Nonwoven Fabric

The silk content ratio (% by mass) in the composite nonwoven fabric was calculated using the following equation:

$$\text{Silk content ratio (\% by mass)} = \{(\text{Basis weight (g/m}^2\text{) of first layer} + \text{Basis weight}$$
$$(\text{g/m}^2\text{) of third layer})/\text{Basis weight (g/m}^2\text{) of composite nonwoven fabric}\} \times 100$$

(4) Thickness (mm) of Composite Nonwoven Fabric

The thickness of the composite nonwoven fabric was measured using five samples that were collected in the same manner as in the measurement of the basis weight (g/m$^2$) of the composite nonwoven fabric. In more detail, for each of the five samples, the thickness was measured at a total of five spots in the center and four corners using a thickness gauge (manufactured by PEACOCK, product number: "R1-250", measuring probe: 25 mm$\varphi$, load: 7 gf/cm$^2$). The arithmetic mean of the thus obtained 25 measured values in total was calculated to determine the average thickness, and this average thickness was defined as the thickness (mm) of the composite nonwoven fabric.

(5) MD Tensile Strength of Composite Nonwoven Fabric

From the composite nonwoven fabric, five rectangular samples of 200 mm (length) × 50 mm (width) were collected. These five samples were collected such that the longitudinal direction of each sample was oriented substantially parallel to the machine direction (MD) of the second layer of the composite nonwoven fabric.
For each of the collected samples, the breaking strength in the longitudinal direction was measured in accordance with JIS L1906 using a tensile tester (AUTOGRAPH AGS-J, manufactured by Shimadzu Corporation) at a chuck distance of 100 mm and a head speed of 300 mm/min. The average of the thus obtained five measured values was calculated and defined as the MD tensile strength of the composite nonwoven fabric at a width of 50 mm.

(6) CD Tensile Strength of Composite Nonwoven Fabric

The CD tensile strength of the composite nonwoven fabric was measured in the same manner as the MD tensile strength of the composite nonwoven fabric, except that the orientation of collecting five samples was changed such that the longitudinal direction of each sample was substantially parallel to the cross direction (CD; i.e. the direction perpendicular to the MD in plan view) of the second layer of the composite nonwoven fabric.

(7) Air Permeability of Composite Nonwoven Fabric (ccs (i.e. cm$^3$/cm$^2$/sec))

From the composite nonwoven fabric, five rectangular samples of 150 mm (length) × 150 mm (width) were collected. These five samples were collected such that the longitudinal direction of each sample was oriented substantially parallel to the machine direction (MD) of the second layer of the composite nonwoven fabric.
For each of the collected samples, the air permeability was measured in accordance with JIS L1906 using a Frazier air permeability tester. The average of the thus obtained five measured values was calculated and defined as the air permeability of the composite nonwoven fabric.

(8) Peel Strength of Composite Nonwoven Fabric

As an index of the strength of the composite nonwoven fabric in the thickness direction, the peel strength of the composite nonwoven fabric was measured as follows.
From the composite nonwoven fabric, five rectangular samples of 200 mm (length) × 20 mm (width) were collected. These five samples were collected such that the longitudinal direction of each sample was oriented substantially parallel to the machine direction (MD) of the second layer of the composite nonwoven fabric.
For each sample, first, the second layer side and the third layer side were peeled off in a region of 50 mm in length from one longitudinal end. Subsequently, the thus peeled second layer side and third layer side were each immobilized on a chuck of a tensile tester (AUTOGRAPH AGS-J, manufactured by Shimadzu Corporation) at a chuck distance of 50 mm. Thereafter, the thus chuck-immobilized second layer side and third layer side were pulled in the opposite direction at a head speed of 100 mm/min to perform T-peeling of the second layer side and the third layer side in the remaining region (a region of 150 mm in length), and the peel strength was measured in this process. The average of the thus obtained five measured values was calculated and defined as the peel strength (N/20-mm width) of the composite nonwoven fabric.

(9) MD Bending Resistance (mm)

As an index of the flexibility of the composite nonwoven fabric in the machine direction (MD), the MD bending resistance was measured as follows.

From the composite nonwoven fabric, five rectangular samples of 200 mm (length) $\times$ 20 mm (width) were collected. These five samples were collected such that the longitudinal direction of each sample was oriented substantially parallel to the machine direction (MD) of the second layer of the composite nonwoven fabric.

For the front side and the back side of each collected sample, the bending resistance (mm) was measured in accordance with JIS L1096:2010 (8.21.1 A method (45° cantilever method)) in a temperature-controlled room having a temperature of 20 $\pm$ 2°C and a humidity of 65 $\pm$ 2%. The average of the thus obtained ten measured values (i.e. values measured on the front side and the back side of the five samples) was calculated and defined as the MD bending resistance (mm).

A larger numerical value of the MD bending resistance means a higher flexibility in the machine direction (MD).

(10) CD Bending Resistance (mm)

As an index of the flexibility of the composite nonwoven fabric in the direction perpendicular to the machine direction in plan view (CD), the CD bending resistance was measured as follows.

That is, the CD bending resistance (mm) was measured in the same manner as the MD bending resistance, except that the orientation of collecting samples was changed such that the longitudinal direction of each sample was substantially parallel to the cross direction (CD) of the second layer of the composite nonwoven fabric.

A larger numerical value of the CD bending resistance means a higher flexibility in the machine direction (MD).

(11) Touch Feel and Fit

The touch feel and fit of the composite nonwoven fabric were evaluated as follows.

From the composite nonwoven fabric, a test piece to be applied to the face was collected.

This test piece had a circular outer shape of 20 cm in diameter. On the test piece, a hole of 3 cm in diameter was made at each of the parts (three spots) corresponding to the eyes and the nose.

Using this test piece, a sensory evaluation in terms of the touch feel and fit was performed by three female subjects in their twenties.

The test piece was immersed in distilled water for 1 hour and removed thereafter, and excess water droplets on the test piece surface were wiped off with a cotton cloth, after which the test piece was applied to the face of each subject, and the subject was then interviewed regarding the touch feel and fit.

The same operations were performed using the crimped silk web of the below-described Comparative Example 2 and the SMS nonwoven fabric (second layer) of the below-described Example 4.

Based on the interview results obtained from the subjects, a rating A of each subject on the touch feel and fit was determined in accordance with the following evaluation criteria.

The average value of the ratings A given by the three subjects was calculated and rounded off to the nearest whole number to determine the rating of the touch feel and fit.

-Evaluation Criteria-

5: The touch feel and fit were equivalent to those of the crimped silk web and maintained for at least 30 minutes.

4: The touch feel and fit were equivalent to those of the crimped silk web (excluding a case corresponding to the rating "5").

3: The touch feel was equivalent to that of the crimped silk web, but the fit was felt inferior to that of the crimped silk web.

2: The touch feel was inferior to that of the crimped silk web but superior to that of the SMS nonwoven fabric.

1: The touch feel was inferior to that of the crimped silk web and equivalent to that of the SMS nonwoven fabric.

(12) Moisture Retention Time During Wearing

As an index of the liquid retainability of the composite nonwoven fabric, the moisture retention time during wearing of the composite nonwoven fabric was measured.

In the above-described evaluation of the touch feel and fit, the time until the test piece came off the face of each subject was measured, and the average of the values measured for the three subjects was calculated as the moisture retention time during wearing of the composite nonwoven fabric.

A longer moisture retention time during wearing of the composite nonwoven fabric means a higher liquid retainability of the composite nonwoven fabric.

[Examples 2 and 3]

The same operations were performed as in Example 1, except that the basis weight of the first layer and that of the third layer were changed as shown in Table 1.

The results thereof are shown in Table 1.

The basis weight was changed by modifying the rotation speed of the roller-type parallel carding machine.

[Example 4]

<Production of Composite Nonwoven Fabric>

(Production of Silk Fiber Webs (First Layer and Third Layer))

In the same manner as in the production of the first layer in Example 1, a crimped silk fiber web having a basis weight of 13 g/m$^2$ and another crimped silk fiber web having a basis weight of 13 g/m$^2$ were obtained as first and third layers.

(Production of Synthetic Fiber Nonwoven Fabric (Second Layer))

An SMS nonwoven fabric (i.e. in this Example, a nonwoven fabric including one spunbond nonwoven fabric layer and at least one melt-blown nonwoven fabric layer arranged between the one spunbond nonwoven fabric layer) was produced in the following manner as a synthetic fiber nonwoven fabric used as a second layer.

The thus obtained SMS nonwoven fabric contained the fibers in the spunbond nonwoven fabric layer and the fibers in the melt-blown nonwoven fabric layer. These fibers were all non-crimped fibers.

Using a propylene homopolymer having an MFR of 60 g/10 min as a raw material, fibers were formed by melt spinning with a spinneret of 0.6 mmφ at a melting temperature of 230°C, and the thus formed fibers were deposited on a collection surface to form a spunbond nonwoven fabric layer S-1 having an average fiber diameter of 16 μm and a basis weight of 6 g/m$^2$.

Next, using a propylene homopolymer having an MFR of 400 g/10 min as a raw material, fibers were formed by a melt-blowing method under the conditions where a molten product of the propylene homopolymer melted at a melting temperature of 280°C was discharged from a spinneret and a hot air of 280°C was blown against the molten product at the discharge port of the spinneret, and the thus formed fibers were deposited on the spunbond nonwoven fabric layer S-1 to form a melt-blown nonwoven fabric layer M-1 having an average fiber diameter of 3 μm and a basis weight of 1 g/m$^2$ on the spunbond nonwoven fabric layer S-1.

Subsequently, a spunbond nonwoven fabric layer S-2 was formed on the melt-blown nonwoven fabric layer M-1 under the same conditions as in the formation of the spunbond nonwoven fabric layer S-1, whereby a laminated body having a layered structure in which the spunbond nonwoven fabric layer S-1, the melt-blown nonwoven fabric layer M-1, and the spunbond nonwoven fabric layer S-2 were arranged in this order was obtained.

Thereafter, the thus obtained laminated body was embossed at an embossing area ratio of 18% and an embossing temperature of 145°C to integrate the spunbond nonwoven fabric layer S-1, the melt-blown nonwoven fabric layer M-1, and the spunbond nonwoven fabric layer S-2, whereby an SMS nonwoven fabric (synthetic fiber nonwoven fabric) having a basis weight of 13 g/m$^2$ was obtained as the second layer.

In Example 4, the basis weight of this SMS nonwoven fabric (synthetic fiber nonwoven fabric) was measured in the same manner as the basis weight of the second layer of Example 1.

As for the basis weight of the spunbond nonwoven fabric layer S-1 and that of the melt-blown nonwoven fabric layer M-1, single-layer nonwoven fabrics were formed under the same conditions as the formation of the respective layers, and the thus formed single-layer nonwoven fabrics were used to measure the basis weight of each layer in the same manner as the basis weight of the second layer of Example 1.

The average fiber diameter of the spunbond nonwoven fabric layer S-1 was measured in the same manner as the average fiber diameter of the second layer of Example 1.

The average fiber diameter of the melt-blown nonwoven fabric layer M-1 was measured by observation under a scanning electron microscope (SU3500 manufactured by Hitachi Ltd.) at a magnification of ×2,000. In more detail, 30 fibers of 8 μm or less in fiber diameter were selected in a field of view, and the fiber diameter (μm) was measured for each of the selected 30 fibers. The average of the thus measured values was calculated to determine the average fiber diameter of the melt-blown nonwoven fabric layer M-1.

(Production of Composite Nonwoven Fabric)

Using the above-obtained first layer, second layer, and third layer, a composite nonwoven fabric was produced in the same manner as in the production of the composite nonwoven fabric in Example 1.

<Measurements and Evaluations>

For the thus obtained composite nonwoven fabric and its materials, the same measurements and evaluations were performed as in Example 1.
The results thereof are shown in Table 1.

[Example 5]

The same operations were performed as in Example 4, except that the basis weight of the first layer and that of the third layer were changed as shown in Table 1.
The results thereof are shown in Table 1.
The basis weight was changed by modifying the rotation speed of the roller-type parallel carding machine.

[Example 6]

<Production of Composite Nonwoven Fabric>

(Production of Silk Fiber Webs (First Layer and Third Layer))

In the same manner as in the production of the first layer and the third layer in Example 1, a silk fiber web having a basis weight of 13 g/m$^2$ and a silk fiber web having a basis weight of 14 g/m$^2$ were obtained as first and third layers, respectively.

(Production of Synthetic Fiber Nonwoven Fabric (Second Layer))

Using a propylene homopolymer having an MFR of 60 g/10 min as a raw material, fibers were formed by melt spinning in accordance with a spunbonding method at a melting temperature of 230°C using a spinneret of 0.6 mmcp, and the thus obtained fibers were deposited on a collection surface, after which the resultant was embossed at an embossing area ratio of 18% and an embossing temperature of 150°C to obtain a spunbond nonwoven fabric (denoted as "SB (single layer)" in Table 1) having an average fiber diameter of 16 $\mu$m and a basis weight of 17 g/m$^2$ as a synthetic fiber nonwoven fabric (second layer).
The fibers in the thus obtained spunbond nonwoven fabric were non-crimped fibers.

(Production of Composite Nonwoven Fabric)

Using the above-obtained first layer, second layer, and third layer, a composite nonwoven fabric was produced in the same manner as in the production of the composite nonwoven fabric in Example 1.

<Measurements and Evaluations>

For the thus obtained composite nonwoven fabric and its materials, the same measurements and evaluations were performed as in Example 1.
The results thereof are shown in Table 1.

[Comparative Examples 1 and 2]

A single-layer crimped silk fiber web was produced in the same manner as in Example 1, except that the basis weight of the first layer was changed as shown in Table 1 and the first layer was used in place of the laminated body obtained by disposing the first layer, the second layer, and the third layer on one another. Using the thus obtained single-layer crimped silk fiber web, the same measurements and evaluations were performed as in

Example 1.

The results thereof are shown in Table 1.

[Comparative Example 3]

An attempt was made to produce a composite nonwoven fabric in the same manner as in Example 1, except that the commercially available silk fibers (A1 SLIVER, average fiber diameter = 12 $\mu$m, manufactured by Hasegawa Corporation Co., Ltd.) (i.e. non-crimped silk fibers) were not subjected to the mechanical crimping operations.
However, a composite nonwoven fabric could not be produced since the first layer, the second layer, and the third layer could not be entangled (i.e. the shape retainability of the composite nonwoven fabric was severely inadequate).

[Comparative Example 4]

An SMS nonwoven fabric was produced in the same manner as in Example 4, except that the second layer was used in place of the laminated body obtained by disposing the first layer, the second layer, and the third layer on one another. Using the thus obtained SMS nonwoven fabric, the same measurements and evaluations were performed as in Example 1.
The results thereof are shown in Table 1.

[Table 1]

| | | | Example 1 | Example 2 | Example 3 | Example 4 | Example 5 | Example 6 | Comparative Example 1 | Comparative Example 2 | Comparative Example 3 | Comparative Example 4 |
|---|---|---|---|---|---|---|---|---|---|---|---|---|
| Material | First layer | Main component | crimped silk | crimped silk | crimped silk | crimped silk | crimped silk | crimped silk | crimped silk | crimped silk | non-crimped silk | - |
| | | Basis weight (gsm) | 13 | 10 | 6.5 | 13 | 6.5 | 13 | 25 | 30 | 13 | - |
| | | Crimp degree | 20% | 20% | 20% | 20% | 20% | 20% | 20% | 20% | 2% | - |
| | Second layer | Material constitution | SB (single layer) | SB (single layer) | SB (single layer) | SMS | SMS | SB (single layer) | - | - | SB (single layer) | SMS |
| | | Main component | composite crimped | composite crimped | composite crimped | non-crimped | non-crimped | non-crimped | - | - | composite crimped | non-crimped |
| | | Basis weight (gsm) | 17 | 17 | 17 | 13 | 13 | 17 | - | - | 17 | 13 |
| | Third layer | Main component | crimped silk | crimped silk | crimped silk | crimped silk | crimped silk | crimped silk | - | - | non-crimped silk | - |
| | | Basis weight (gsm) | 14 | 10 | 6.5 | 13 | 6.5 | 14 | - | - | 14 | - |
| | | Crimp degree | 20% | 20% | 20% | 20% | 20% | 20% | - | - | 2% | - |

| Composite nonwoven fabric | | Example 1 | Example 2 | Example 3 | Example 4 | Example 5 | Example 6 | Comparative Example 1 | Comparative Example 2 | Comparative Example 3 | Comparative Example 4 |
|---|---|---|---|---|---|---|---|---|---|---|---|
| | Entangling method | spunlace | spunlace | spunlace | spunlace | spunlace | spunlace | spunlace | spunlace | spunlace | spunlace |
| | Basis weight (gsm) | 44 | 37 | 30 | 39 | 26 | 44 | 25 | 30 | | 13 |
| | Silk content ratio (% by mass) | 61 | 54 | 43 | 67 | 50 | 61 | 100 | 100 | | 0 |
| | Thickness | 0.44 mm | 0.43 mm | 0.39 mm | 0.31 mm | 0.27 mm | 0.40 mm | 0.25 mm | 0.30 mm | | 0.23 mm |
| | MD tensile strength (N/50 mm) | 36 | 29 | 25 | 36 | 29 | 39 | 16 | 19 | | 25 |
| | CD tensile strength (N/50 mm) | 13 | 10 | 8 | 11 | 11 | 15 | 3 | 4 | | 12 |
| | Air permeability | 150 ccs | 171 ccs | 189 ccs | 103 ccs | 118 ccs | 124 ccs | 343 ccs | 286 ccs | (could not be entangled) | 225 ccs |
| | Peel strength | 0.8 N | 0.9 N | 0.7 N | 1.3 N | 1.0N | 0.5 N | - | - | | - |
| | Flexibility (MD bending resistance) | 44 mm | 42 mm | 41 mm | 43 mm | 42 mm | 45 mm | 36 mm | 37 mm | | 44 mm |
| | Flexibility (CD bending resistance) | 25 mm | 23 mm | 21 mm | 25 mm | 22 mm | 26 mm | 19 mm | 20 mm | | 32 mm |
| | Touch feel and fit | 5 | 5 | 5 | 3 | 3 | 5 | 5 | 5 | | 1 |
| | Liquid retainability (Moisture retention time during wearing) | 30 minutes or longer | 30 minutes or longer | 30 minutes or longer | 23 minutes | 21 minutes | 30 minutes or longer | 30 minutes or longer | 30 minutes or longer | | 1 minute |

EP 4 148 175 A1

As shown in Table 1, the composite nonwoven fabrics of Examples 1 to 6, which included a silk region containing crimped silk fibers as a main component and a synthetic fiber nonwoven fabric region containing polyolefin resin-containing synthetic fibers as a main component, were excellent in flexibility, strength (i.e. MD tensile strength, CD tensile strength, and peel strength), and liquid retainability.

In contrast to these Examples, the composite nonwoven fabrics of Comparative Examples 1 and 2, which did not include a synthetic fiber nonwoven fabric region, were insufficient in strength.

In addition, the composite nonwoven fabric of Comparative Example 4, which did not include a silk region, was insufficient in liquid retainability and flexibility.

Further, as opposed to Example 1, in Comparative Example 3 where non-crimped silk fibers were used in place of crimped silk fibers, a composite nonwoven fabric could not be produced since the layers could not be entangled.

Among Examples 1 to 6, in Examples 1 to 3 where the synthetic fibers contained crimped fibers and Examples 4 and 5 where the synthetic fiber nonwoven fabric region was derived from an SMS nonwoven fabric, the composite nonwoven fabrics had a higher peel strength.

The disclosure of Japanese Patent Application No. 2020-116943 filed on July 7, 2020 is hereby incorporated by reference in its entirety.

All the documents, patent applications, and technical standards that are described in the present specification are hereby incorporated by reference to the same extent as if each individual document, patent application, or technical standard is concretely and individually described to be incorporated by reference.

## Claims

1. A composite nonwoven fabric, comprising:

    a silk region that comprises crimped silk fibers as a main component; and
    a synthetic fiber nonwoven fabric region that comprises polyolefin resin-containing synthetic fibers as a main component.

2. The composite nonwoven fabric according to claim 1, having a thickness of 1.00 mm or less.

3. The composite nonwoven fabric according to claim 1 or 2, having a basis weight of from 20 g/cm$^2$ to 60 g/cm$^2$.

4. The composite nonwoven fabric according to any one of claims 1 to 3, wherein a content ratio of the silk fibers with respect to a total mass of the composite nonwoven fabric is from 30% by mass to 90% by mass.

5. A spunlace nonwoven fabric, comprising the composite nonwoven fabric according to any one of claims 1 to 4.

6. The composite nonwoven fabric according to any one of claims 1 to 5, having a tensile strength of 20 N/50 mm or more in a machine direction.

7. The composite nonwoven fabric according to any one of claims 1 to 6, having a tensile strength of 5 N/50 mm or more in a direction perpendicular to the machine direction in plan view.

8. The composite nonwoven fabric according to any one of claims 1 to 7, wherein the polyolefin resin comprises a polypropylene resin.

9. The composite nonwoven fabric according to any one of claims 1 to 8, wherein the synthetic fibers have an average fiber diameter of from 10 μm to 30 μm.

10. The composite nonwoven fabric according to any one of claims 1 to 9, wherein the synthetic fibers comprise crimped fibers.

11. The composite nonwoven fabric according to any one of claims 1 to 8, wherein the synthetic fiber nonwoven fabric region is derived from an SMS nonwoven fabric that comprises two spunbond nonwoven fabrics as outermost layers and at least one melt-blown nonwoven fabric arranged between the two spunbond nonwoven fabrics.

12. A method of producing the composite nonwoven fabric according to any one of claims 1 to 11, the method comprising:

the step of preparing a silk fiber web that comprises crimped silk fibers as a main component;
the step of preparing a synthetic fiber nonwoven fabric that comprises polyolefin resin-containing synthetic fibers as a main component; and
the step of obtaining a composite nonwoven fabric by entangling the silk fiber web with the synthetic fiber nonwoven fabric.

13. The method according to claim 12, wherein, in the step of obtaining a composite nonwoven fabric, the silk fiber web with the synthetic fiber nonwoven fabric are entangled by a spunlacing process.

## INTERNATIONAL SEARCH REPORT

| | International application No. |
|---|---|
| | PCT/JP2021/025303 |

A.    CLASSIFICATION OF SUBJECT MATTER
D04H 1/4266(2012.01)i; D04H 1/498(2012.01)i; D04H 3/16(2006.01)i; D04H
5/03(2012.01)i
FI: D04H1/4266; D04H1/498; D04H3/16; D04H5/03
According to International Patent Classification (IPC) or to both national classification and IPC

B.    FIELDS SEARCHED

Minimum documentation searched (classification system followed by classification symbols)
D04H1/4266; D04H1/498; D04H3/16; D04H5/03

Documentation searched other than minimum documentation to the extent that such documents are included in the fields searched
|  |  |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

C.    DOCUMENTS CONSIDERED TO BE RELEVANT

| Category* | Citation of document, with indication, where appropriate, of the relevant passages | Relevant to claim No. |
|---|---|---|
| Y | JP 6-235154 A (HIRO INTERNATL KK) 23 August 1994 (1994-08-23) claim 1, paragraphs [0013], [0020], [0021], fig. 1 | 1-4, 8, 12 |
| A | claim 1, paragraphs [0013], [0020], [0021], fig. 1 | 5-7, 9-11, 13 |
| Y | JP 8-3855 A (UNITIKA LTD.) 09 January 1996 (1996-01-09) claim 1, paragraph [0010] | 1-4, 8, 12 |
| A | claim 1, paragraph [0010] | 5-7, 9-11, 13 |
| A | CN 107974766 A (QINGDAO UNIVERSITY) 01 May 2018 (2018-05-01) entire text, all drawings | 1-13 |
| A | CN 105113121 A (CHANGXING SANMASS TEXTILE CO., LTD.) 02 December 2015 (2015-12-02) entire text, all drawings | 1-13 |

☒    Further documents are listed in the continuation of Box C.          ☒    See patent family annex.

| | |
|---|---|
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| 19 August 2021 (19.08.2021) | 31 August 2021 (31.08.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2021/025303 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | CN 101612416 A (HENGAN (FUJIAN) HOLDING GROUP CO., LTD.) 30 December 2009 (2009-12-30) entire text | 1-13 |
| A | JP 2010-222726 A (EMU EMU EEMU KK) 07 October 2010 (2010-10-07) entire text, all drawings | 1-13 |
| A | JP 2006-207069 A (YUHO CO., LTD.) 10 August 2006 (2006-08-10) entire text | 1-13 |
| A | JP 2018-100469 A (SHINSHU UNIVERSITY) 28 June 2018 (2018-06-28) entire text, all drawings | 1-13 |

Form PCT/ISA/210 (continuation of second sheet) (January 2015)

**INTERNATIONAL SEARCH REPORT**

Information on patent family members

| International application No. |
| --- |
| PCT/JP2021/025303 |

| Patent Documents referred in the Report | Publication Date | Patent Family | Publication Date |
| --- | --- | --- | --- |
| JP 6-235154 A | 23 Aug. 1994 | (Family: none) | |
| JP 8-3855 A | 09 Jan. 1996 | (Family: none) | |
| CN 107974766 A | 01 May 2018 | (Family: none) | |
| CN 105113121 A | 02 Dec. 2015 | (Family: none) | |
| CN 101612416 A | 30 Dec. 2009 | (Family: none) | |
| JP 2010-222726 A | 07 Oct. 2010 | (Family: none) | |
| JP 2006-207069 A | 10 Aug. 2006 | (Family: none) | |
| JP 2018-100469 A | 28 Jun. 2018 | CN 108221082 A entire text, all drawings KR 10-2018-0072536 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9707273 A
- JP 2003105661 A
- JP 2006207069 A
- JP 4009196 B
- JP 5139669 B
- JP 5289459 B
- WO 2020036143 A
- JP 2020116943 A